**Europäisches Patentamt**

**European Patent Office**

**Office européen des brevets**

(19)

(11) Veröffentlichungsnummer: **0 019 741 B1**

(12) **EUROPÄISCHE PATENTSCHRIFT**

(45) Veröffentlichungstag der Patentschrift:
16.10.85

(21) Anmeldenummer: 80102364.9

(22) Anmeldetag: 01.05.80

(51) Int. Cl.⁴: **G 01 N 33/546**, G 01 N 33/68

(54) Latex-Reagenz, Verfahren zu seiner Herstellung, seine Verwendung und ein das Reagenz enthaltendes Mittel.

(30) Priorität: 07.05.79 DE 2918342

(43) Veröffentlichungstag der Anmeldung:
10.12.80 Patentblatt 80/25

(45) Bekanntmachung des Hinweises auf die Patenterteilung:
16.10.85 Patentblatt 85/42

(84) Benannte Vertragsstaaten:
AT BE CH DE FR GB IT LI LU NL SE

(56) Entgegenhaltungen:
EP - A - 0 000 772
DE - A - 2 749 956
DE - A - 2 754 645
US - A - 3 658 982
US - A - 4 143 203

Journal of Immunological Methods, 22 (1978) pp. 247-251
G. Virella "Nephelometric Method for Determination of Rheumatoid Factor"

(73) Patentinhaber: **BEHRINGWERKE AKTIENGESELLSCHAFT, Postfach 1140, D-3550 Marburg/Lahn (DE)**

(72) Erfinder: **Kapmeyer, Wolfgang, Dr., Friedhofstrasse 11, D-3550 Marburg/Lahn (DE)**
Erfinder: **Sieber, Axel, Dr., Sonnenhang 25, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

**Beschreibung**

Die Erfindung betrifft ein Latex-Reagenz, seine Herstellung und Verwendung zum Nachweis und zur Bestimmung von klinisch relevanten Proteinen und Peptiden mit nephelometrischen oder turbidimetrischen Methoden.

Neben Radio- und Enzymimmunoassays werden auch nephelometrische Methoden zur Bestimmung von Antigenen und Antikörpern herangezogen. Die Lichtstreuung und somit die Empfindlichkeit der nephelometrischen Methode kann durch die Partikelgrösse des Antigens bzw. des Antikörpers verstärkt werden. Eine brauchbare Methode besteht darin, das Antigen oder den Antikörper an Latexpartikel, die selbst das Licht streuen, zu binden.

Wie aus den Arbeiten von J. Grange und Mitarbeitern in J. Immunol. Meth. 18 (1977), 365–375 hervorgeht, nimmt bei der Titration eines an Latex gebundenen Antikörpers mit Antigen zunächst einmal das Streulichtsignal ab, weil mit zunehmender Agglutination die Teilchenzahl abnimmt. Danach lässt ein weiterer Anstieg der Antigen-Konzentration die Aggregatgrösse ansteigen, womit die Streulichtintensität zunimmt, bis bei weiterem Anstieg der Antigenmenge im Antigenüberschuss das Streulichtsignal wieder abnimmt. Für den quantitativen Test ist es wichtig, die Agglutinationsbedingungen des Latex so zu kontrollieren, dass bei einer Titration von Latex-gebundenen Antikörpern mit Antigen oder von Latexgebundenem Antigen mit Antikörpern die Zunahme der Agglutination in weitem Bereich als Zunahme der Streulichtintensität gemessen werden kann.

Eine wichtige Gruppe von Antikörpern sind die Rheumafaktoren. Als Rheumafaktoren bezeichnet man Anti-Immunglobuline, gerichtet gegen teilweise veränderte Immunglobuline der Klassen IgG oder IgM. Die Rheumafaktoren gehören selbst zu den Immunglobulinklassen IgM, IgG und IgA. Testmethoden zum Nachweis von rheumatoiden Faktoren beruhen auf der Agglutination von beispielsweise Erythrozyten oder Latexpartikeln, die mit IgG beschichtet sind. Diese Verfahren erlauben nur eine qualitative Bestimmung von Rheumafaktoraktivitäten, und die Ablesung der Ergebnisse dieser Agglutinations-Tests ist sehr subjektiv.

Die von G. Virella und Mitarbeitern in J. Immunol. Meth. 22 (1978) 247–251 beschriebene Methode dokumentiert den bisherigen Stand der Technik.

Verfahren und Reagenzien zur Bestimmung eines Partners einer immunlogischen Reaktion mit Hilfe von Latices eines Grössenbereiches sind auch in der DE-A-2 749 956 und der EP-A-772 beschrieben. Nachteilig bei allen Verfahren und Reagenzien des Standes der Technik sind ein nicht eindeutiger Verlauf der Streulichtintensität bei der Titration, ein sehr schmaler Messbereich, d.h. ein steiler Anstieg der Referenzkurve sowie hohe Blindwerte des Latexreagenzes.

Es wurde nun überraschend gefunden, dass diese Nachteile dadurch vermieden werden können, dass ein Latexreagenz verwendet wird, das Latexpartikel von 0,1 bis 0,35 µm, die mit der 0,6- bis 3,3-fachen Gewichtsmenge des korrespondierenden Antigens oder Antikörpers beladen sind, und Latexpartikel von 0,5 bis 2 µm, die mit etwa der 0,1-fachen Gewichtsmenge desselben Antigens oder Antikörpers beladen sind, im Volumenverhältnis von 4 : 1 bis 1 : 2 enthält.

Die Erfindung betrifft auch ein Verfahren zur Herstellung dieses Latex-Reagenzes gemäss Anspruch 2, ein diagnostisches Mittel enthaltend dieses Reagenzes gemäss Anspruch 3 und die Verwendung dieses Reagenzes gemäss Anspruch 5.

Die eine Komponente des Reagenzes besteht aus einer Suspension von Latexpartikeln in der Grösse von 0,1 µm bis 0,35 µm, vorzugsweise 0,15 bis 0,25 µm. Diese Partikel sind mit 100 bis 500 mg human IgG pro 150 mg Latex, vorzugsweise mit 150 mg Human-IgG pro 150 mg Latex beladen. Die zweite Komponente des Reagenzes besteht aus einer Suspension von Latexpartikeln in der Grösse von 0,5 µm bis 2 µm, vorzugsweise 0,5 µm bis 0,8 µm, wie es z.B. als Latex-Rheumafaktoren-Reagenz zur visuellen Agglutinationstechnik im Handel ist. Dieses Latex-Rheumafaktoren-Reagenz ist nach herkömmlichen Methoden mit 0,1 mg IgG pro mg Latex beladen. Es kann selbstverständlich wie Komponente 1 auch selbst hergestellt werden. Die beiden Komponenten werden im Volumenverhältnis von etwa 4 : 1 bis etwa 1 : 2 gemischt. Die Mischung beider Einzelkomponenten, die vorzugsweise als wässrige Suspension vorliegen, die Puffersubstanzen, Neutralsalze, Stabilisatoren und Konservierungsmittel enthalten kann, wird ultraschallbehandelt. Gegebenenfalls wird etwa eine Woche bei Raumtemperatur inkubiert. Dadurch wird eine Readsorption von gegebenenfalls durch die Ultraschallbehandlung vom Latex abgetrennten IgG bewirkt. Das auf diese Weise erhaltene Latexreagenz zeichnet sich durch einen grossen Messbereich aus. Gegebenenfalls kann daraus ein Trockenpulver bereitet werden, das zum Gebrauch resuspendiert werden kann.

Das erfindungsgemässe Latexreagenz kann verwendet werden in der Nephelometrie und Turbidimetrie sowie im Particle Counting Immuno Assay (PACIA) zum Nachweis und zur Bestimmung von Substanzen wie proteinartigen Antigenen und Haptenen, Antikörpern, Virusbestandteilen, Zellbestandteilen, Enzymen und Hormonen. Bevorzugt ist die Verwendung des Latexreagenzes zur Bestimmung von Plasmaproteinen. Besonders geeignet ist das Latexreagenz zur Bestimmung von Spurenproteinen, die aufgrund von geringen Konzentrationen im Serum schwierig mit anderen Methoden nachweisbar sind. Noch besser ist das Latexreagenz zur nephelometrischen Bestimmung von Rheumafaktoren, von Immunkomplexen und von schwangerschaftsspezifischen Proteinen wie beispielsweise schwangerschaftsspezifischem $\beta_1$-Glykoprotein, schwanger-

schaftassoziiertem alpha$_2$-Glykoprotein und humanem Plazenta-Lactogen geeignet.

Das Latexreagenz ist brauchbar zur Bestimmung von Proteinen und Peptiden sowohl nach kinetischen als auch Endpunktmethoden.

Die Latexpartikel können adsorptiv oder durch kovalente Bindung von Proteinen beladen werden. Zur kovalenten Bindung können Latexderivate mit den freien Carboxylgruppen, Aminogruppen, Aldehydgruppen und Epoxygruppen sowie vicinalen Hydroxylgruppen herangezogen werden. Weiterhin können kleinere Peptide, wie Hormone, auch über ein Trägerprotein an das Latex gebunden werden. Am günstigsten erwies sich die adsorptive Bindung von Proteinen an das Latex.

Die zur Herstellung des erfindungsgemässen Reagenzes verwendbaren Latices können beispielsweise durch Polymerisation olefinisch ungesättigter Monomere erhalten werden. Bevorzugt sind Styrol-Copolymerisate, wie Styrol-Butadien-Copolymerisate und Acrylnitril-Butadien-Styrol-Copolymerisate, Vinylacetat-Acrylat-Copolymerisate oder Vinylchlorid-Acrylat-Copolymerisate.

Polystyrol-Latex-Suspensionen oder Emulsionen geeigneter Teilchengrösse können auch unter verschiedenen Handelsbezeichnungen von einer Anzahl von Herstellern erhalten werden, beispielsweise Lytron®-Latices von der Firma Monsanto Company, St. Louis, Missouri, Dylex®-Latices von der Firma Sinclair-Koppers Company, Pittsburgh, Pennsylvania, und Dow-Latex-Particles von der Firma Dow Chemical Company, Indianapolis, Indiana, sowie Acrylpolymerisate, wie Acryl®-Teilchen von der Firma Colab Laboratories, Inc., Chicago Heights, Illinois (Polymethacrylsäureester), und Ubatol U-7001 von der Firma Stanley Chemical, Cambridge, Massachusetts.

Die Erfindung wird anhand eines Ausführungsbeispieles näher beschrieben.

1) Herstellung des Latexreagenzes

150 mg nach bekannten Methoden hergestelltes Polystyrol-Latex mit einer Teilchengrösse von 0,1 bis 0,35 μm, vorzugsweise 0,15 bis 0,25 μm, wird in einer Konzentration von 3,8 mg Latex pro ml in einem 0,1 M Glycin und 0,17 M Natriumchlorid enthaltenen Puffer pH 8,2 suspendiert. Mit 150 mg Human-IgG (10 min bei 60 °C hitzeaggregiert) und 920 mg Human-Albumin pro 150 mg Latex wird für 5 Stunden bei Raumtemperatur gerührt und dann mit physiologischer Kochsalzlösung, die 2 mg/ml Rinderserumalbumin enthält, auf 0,5 mg Latex pro ml verdünnt. Man erhält so ein mit aggregiertem IgG hochbeladenes Latexpräparat, welches allerdings für sich noch nicht für eine lasernephelometrische Bestimmung geeignet ist.

Weiterhin wird handelsübliches Latex-Rheumafaktoren-Reagenz zur Bestimmung von Rheumafaktoren nach der visuellen Agglutinationstechnik (Behringwerke) ebenfalls mit physiologischer Kochsalzlösung (2 mg/ml Rinderserumalbumin) auf 0,5 mg Latex pro ml verdünnt. Das mit IgG hochbeladene Latexpräparat wird mit handelsüblichem Latex-Rheumafaktoren-Reagenz im Volumenverhältnis 4 : 1 bis 0,5 : 1, vorzugsweise 2 : 1, zusammengegeben und je 250 ml Ansatz für 20 Sekunden ultraschallbehandelt. Das Reagenz wird dann eine Woche bei Raumtemperatur inkubiert und anschliessend bei +4 °C gelagert.

2) Nephelometrische Bestimmung des Rheumafaktors

Ein Rheumafaktoren-Referenz-Serum, das ein Substandard des WHO Rheumafaktoren-Standards ist und 150 IU/ml Rheumafaktoren-Aktivität besitzt, sowie die zu untersuchenden Seren werden 30 min auf 56 °C zur Komplementinaktivierung erhitzt. Das Referenz-Serum wird in einer geometrischen Reihe von 1 : 6 bis 1 : 768 verdünnt. Das ergibt Rheumafaktoren-Aktivitäten von 25 bis 0,2 IU/ml in diesen Verdünnungen. Die Seren werden 1 : 100 verdünnt. 50 μl Serum- oder Referenz-Serum-Verdünnung und 75 μl des gut aufgeschüttelten Latex-Reagenzes werden in LN-Küvetten (Sarstedt) gemischt.

Nach 1 Stunde Inkubationszeit werden 100 μl physiologische Kochsalzlösung mit 0,1 M Borat pH 8,1 hinzugegeben, und nach weiteren 15 min wird die Lichtstreuung im Laser-Nephelometer gemessen.

Zur Auswertung werden die für die Standardverdünnungen erhaltenen Voltwerte in einer Referenzkurve auf halblogarithmischem Papier aufgetragen. Anhand der Referenzkurve werden die Konzentrationen an Rheumafaktoren-Aktivität für Patientenserum ermittelt.

Der hier beschriebene Test erlaubt eine Bestimmung von Rheumafaktoren-Aktivitäten in einem Bereich zwischen 0,2 und 25 IU/ml für unverdünntes Serum. Bei einer Verdünnung der Seren von 1 : 100 ist somit eine Rheumafaktoren-Bestimmung zwischen 20 und 2500 IU/ml Rheumafaktoren-Aktivität im Serum möglich. Dieses ist der für die Diagnose von Rheumaerkrankungen wichtige Messbereich. Der beschriebene Test zeigt eine gute Reproduzierbarkeit in der Serie VK = 3% sowie von Tag zu Tag VK = 7%.

Entsprechend dem Ausführungsbeispiel können andere Antikörper oder Antigene bestimmt werden.

**Patentansprüche**

1. Ein Latex-Reagenz zum Nachweis und zur Bestimmung von Antikörpern oder Antigenen, dadurch gekennzeichnet, dass es Latexpartikeln von 0,1 bis 0,35 μm Durchmesser, die mit der 0,6- bis 3,3-fachen Gewichtsmenge des korrespondierenden Antigens oder des korrespondierenden Antikörpers beladen sind, und von Latexpartikeln von 0,5 bis 2 μm, die mit etwa der 0,1-fachen Gewichtsmenge desselben Antigens oder Antikörpers beladen sind, im Volumenverhältnis von 4 : 1 bis 1 : 2 enthält.

2. Verfahren zur Herstellung eines Latex-Reagenzes zum Nachweis und zur Bestimmung von Antikörpern oder Antigenen, dadurch gekennzeichnet, dass man eine Suspension von Latexpartikeln von 0,1 bis 0,35 μm Durchmesser mit der

0,6- bis 3,3-fachen Gewichtsmenge des korrespondierenden Antigens oder des korrespondierenden Antikörpers reagieren lässt, diese Suspension mit einer Suspension von Latexpartikeln von 0,5 bis 2 µm, die mit etwa der 0,1-fachen Gewichtsmenge desselben Antigens oder Antikörpers beladen sind, im Volumenverhältnis von 4 : 1 bis 1 : 2 mischt und gegebenenfalls ultraschallbehandelt und gegebenenfalls stehen lässt und gegebenenfalls daraus ein Trockenpulver bereitet.

3. Diagnostisches Mittel, enthaltend ein Reagenz nach Anspruch 1 in einer flüssigen Phase sowie gegebenenfalls Puffersubstanzen, Stabilisatoren, Neutralsalze und/oder Konservierungsmittel.

4. Diagnostisches Mittel nach Anspruch 3, enthaltend mit Immunglobulin G beladene Latexpartikel, zur Bestimmung von Rheumafaktoren.

5. Verwendung des Reagenzes nach Anspruch 1 zur Bestimmung von Antigenen oder Antikörpern.

6. Verfahren zum Nachweis und zur Bestimmung eines Antigens oder Antikörpers, dadurch gekennzeichnet, dass ein Reagenz nach Anspruch 1, welches mit dem korrespondierenden Antikörper beziehungsweise Antigen beladen ist, in flüssiger Phase mit dem zu bestimmenden Antigen oder Antikörper zusammengebracht und die Lichtstreuung gemessen wird.

## Claims

1. A latex reagent for detecting and determining antibodies or antigens comprising latex particles having a diameter of from 0.1 to 0.35 µm which are loaded with a 0.6 to 3.3-fold amount by weight of the corresponding antigen or the corresponding antibody, and latex particles having a diameter of from 0.5 to 2 µm, which are loaded with about the 0.1-fold amount by weight of the same antigen or antibody, in a ratio of from 4 : 1 to 1 : 2.

2. Process for preparing a latex reagent for detecting and determining of antibodies or antigens, which comprises reacting a suspension of latex particles having a diameter of from 0.1 to 0.35 µm with the 0.6 to 3.3-fold amount by weight of the corresponding antigen or the corresponding antibody, mixing this suspension with a suspension of latex particles having a diameter of from 0.5 to 2 µm which are loaded with about the 0.1-fold amount by weight of the same antigen or antibody, in a ratio by volume of from 4 : 1 to 1 : 2, and optionally a treatment with supersonics and optionally an incubation and optionally preparation of a dry form.

3. Diagnostic agent comprising a reagent as claimed in Claim 1 in liquid phase and optionally buffer substances, stabilizers, neutral salts and/or preserving agents.

4. Diagnostic agent as claimed in Claim 3 comprising latex particles loaded with immunglobulin G for the determination of rheumatoid factors.

5. Use of the reagent as claimed in Claim 1 for determination of antigens or antibodies.

6. A process for detecting and determining an antigen or antibody, which comprises contacting a reagent as claimed in Claim 1 loaded with the corresponding antibody or antigen, respectively, in liquid phase with the antigen or antibody to be determined, and measuring the scattered light.

## Revendications

1. Réactif au latex pour la détection et la détermination d'anticorps ou d'antigènes, caractérisé en ce qu'il contient des particules de latex de 0,1 à 0,35 µm de diamètre, qui sont chargées avec de 0,6 à 3,3 fois la quantité pondérale de l'antigène correspondant ou de l'anticorps correspondant, et des particules de latex de 0,5 à 2 µm qui sont chargées avec environ 0,1 fois la quantié pondérale de ce même antigène ou anticorps, dans un rapport volumique de 4 : 1 à 1 : 2.

2. Procédé de préparation d'un réactif au latex pour la détection et la détermination des anticorps et des antigènes, caractérisé en ce qu'on fait réagir une suspension de particules de latex de 0,1 à 0,35 µm de diamètre avec de 0,6 à 3,3 fois la quantité pondérale de l'antigène correspondant ou de l'anticorps correspondant, on mélange cette suspension avec une suspension de particules de latex de 0,5 à 2 µm, chargée avec environ 0,1 fois la quantité pondérale du même antigène ou anticorps, dans un rapport volumique de 4 : 1 à 1 : 2 et éventuellement on traite par les ultrasons, on laisse éventuellement au repos et on convertit éventuellement sous une forme pulvérulente.

3. Agent de diagnostic, contenant un réactif selon la revendication 1 dans une phase liquide, ainsi qu'éventuellement des substances tampons, des stabilisants, des sels neutres et/ou des agents de conservation.

4. Agent de diagnostic selon la revendication 3, contenant des particules de latex chargées avec une immunoglobuline G, pour la détermination des facteurs rhumatoïdes.

5. Utilisation du réactif selon la revendication 1 pour la détermination des antigènes ou des anticorps.

6. Procédé pour la détection et la détermination d'un antigène ou d'un anticorps, caractérisé en ce qu'on met dans une phase liquide un réactif selon la revendication 1, chargé avec l'anticorps ou l'antigène correspondant, avec l'antigène ou l'anticorps à déterminer et on mesure la dispersion de la lumière.